(19) Europäisches Patentamt
European Patent Office
Office européen des brevets

(11) **EP 1 852 702 B1**

(12) **EUROPEAN PATENT SPECIFICATION**

(45) Date of publication and mention
of the grant of the patent:
**18.11.2009 Bulletin 2009/47**

(51) Int Cl.:
***G01N 33/569*** *(2006.01)* ***C12N 5/00*** *(2006.01)*

(21) Application number: **06009364.8**

(22) Date of filing: **05.05.2006**

(54) **Method for quantifying a cell population of interest contained in a human blood sample**

Verfahren zur Quantifizierung einer bestimmten Zellpopulation in einer menschlichen Blutprobe

Procédé de quantification d'une population cellulaire d'intérêt contenu dans un échantillon de sang humain

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**07.11.2007 Bulletin 2007/45**

(73) Proprietor: **Fraunhofer-Gesellschaft zur Förderung der angewandten Forschung e.V. 80686 München (DE)**

(72) Inventors:
• **Wurth, Rainer**
**04103 Leipzig (DE)**
• **Bold, Angelika**
**04317 Leipzig (DE)**
• **Sack, Ulrich**
**04275 Leipzig (DE)**

(74) Representative: **Vossius & Partner Siebertstraße 4 81675 München (DE)**

(56) References cited:
**US-A- 5 840 502 US-A1- 2003 147 886**

• **[Online] XP002408216 Retrieved from the Internet: URL:http://www.stemcell.com/ technical/1502 2_15062-PIS.pdf>**
• **[Online] XP002408217 Retrieved from the Internet: URL:http://www.stemcell.com/ technical/1502 3_15063-PIS.pdf>**
• **AVENT N D: "HUMAN ERYTHROCYTE ANTIGEN EXPRESSION: ITS MOLECULAR BASES" BRITISH JOURNAL OF BIOMEDICAL SCIENCE, ROYAL SOCIETY OF MEDICINE SERVICES, LONDON, GB, vol. 54, no. 1, March 1997 (1997-03), pages 16-37, XP001118159 ISSN: 0967-4845**

**EP 1 852 702 B1**

**Description**

The present invention relates to a method for quantifying a cell population of interest contained in a human blood sample, comprising the steps of: contacting a sample of human blood with a cocktail comprising a first component, comprising at least one non-human antibody, antibody fragment or derivative thereof, capable of specifically binding to an antigen expressed on the surface of a first cell population distinct from a second cell population, said second cell population being the cell population of interest and being devoid of said antigen, complexed to a second component, comprising at least one non-human antibody, antibody fragment or derivative thereof, capable of specifically binding to the surface of human erythrocytes; separating antibody-labelled from unlabelled cells contained in the blood sample; collecting the supernatant comprising the second cell population; concentrating the second cell population contained in the supernatant and resuspending said cell population in a liquid capable of selectively lysing erythrocytes; concentrating the obtained cells, essentially removing all supernatant and resuspending said cells in a defined volume of physiological buffer; and determining the number of the cells of the second cell population contained in the sample.

Counts of various types of blood cells are often indicative of a disease. For example extremely high numbers of B cells in the blood can indicate a B cell lymphoma such as Non-Hodgkin-lymphoma. Leukocytosis (especially of neutrophile granulocytes) often indicates various infections; acidocytosis can indicate scarlet fever, measles, gonorrhea, leprosy, dysentery or allergy or worms; lymphocytosis can indicate pertussis, rubella, mumps. Similarly, the absolute $CD4^+$ T-lymphocyte count (CD4 count) serves as the major clinical indicator of immunocompetence in patients with HIV infection (U.S.Department of Health and Human Services 2005), because the $CD4^+$ T-cell is the natural target host for the HIV-virus. It is considered to be the best surrogate marker for the monitoring of the clinical course of the infection with HIV, as these cells are the natural host to the virus. The CD4 count is used for the assessment of the degree of immune deterioration and the speed of progression towards AIDS, furthermore to decide when to initiate antiretroviral therapy (ART) and opportunistic infection prophylaxis (OI), and also for the monitoring of the immunologic response to ART when viral load measurements are unavailable (World Health Organization 2005).

Flow cytometry has been the accepted standard reference method for enumerating the $CD4^+$ T-lymphocytes since the early 1980s (Donnenberg and Donnenberg 2004). However, this method is prevented from being widely used in resource-limited areas by two factors: First, flow cytometry requires highly expensive laboratory equipment and reagents as well as specially trained staff for running the tests. Conventional flow cytometers, such as FACSCalibur™ or Coulter systems, cost US $50,000 or more to set up and about US $20 to US $30 for each test (aidsmap 2005). Second, equipment maintenance is often difficult due to frequent electrical power failures which cause machine-related problems. Various investigators have tried to reduce the costs of flow cytometry (Chianese et al. 2003; Janossy et al. 2002; Pattanapanyasat et al. 2003; Storie et al. 2003a; Storie et al. 2003b), but the procedure of monitoring costs still an amount to approx. US $10 per sample. Recently developed portable flow cytometers are advertised by the manufacturer to run at a cost of US $2 per sample (CyTecs GmbH 2005).

During the last years, a number of research groups have developed lower-cost and less technically demanding immunologic assays for the enumeration of the $CD4^+$ T-lymphocytes, which cost approx. US $1-3 per sample. These tests are microscopy-based, and use positive selection methods such as Dynabeads® by Dynal Biotech (Bi et al. 2005; Diagbouga et al. 2003) or Cyto-Spheres by Beckman-Coulter (Balakrishnan et al. 2004; Carella et al. 1995). However, until today, none of these methods has become generally established in resource-limited areas (Crowe et al. 2003). Moreover, there is also no practical test available to monitor cell numbers of cell populations other than $CD4^+$ T-lymphocytes in human blood samples with acceptable costs and suitable requirement of technical equipment and laboratory experience.

Thus, the technical problem underlying the present invention was to provide means and methods for the improvement of the analysis of cell populations in samples taken from a subject, in particular in terms of costs and handling in resource-limited situations.

The solution to this technical problem is achieved by providing the embodiments characterized in the claims.

Accordingly, the present invention relates to a method for quantifying a cell population of interest contained in a human blood sample, comprising the steps of:

   (a) contacting a sample of human blood with a cocktail comprising

      (i) a first component, comprising at least one non-human antibody, antibody fragment or derivative thereof, capable of specifically binding to an antigen expressed on the surface of a first cell population distinct from a second cell population, said second cell population being the cell population of interest and being devoid of said antigen, wherein said second cell population is selected from the group consisting of the $CD4^+$ cell population and the $CD8^+$ cell population complexed to
      (ii) a second component, comprising at least one non-human antibody, antibody fragment or derivative thereof, capable of specifically binding to the surface of human erythrocytes;

2

wherein the contacting is performed under conditions that allow labelling of antigens with their cognate antibody;

(b) separating antibody-labelled from unlabelled cells contained in the blood sample by means of density centrifugation wherein the minimal degree of density of the medium is about 1.077 g/mL;
(c) collecting the entire supernatant comprising the second cell population;
(d) concentrating the second cell population contained in the supernatant and resuspending said cell population in a liquid capable of selectively lysing erythrocytes;
(e) washing cells obtained in step (d) in a physiological buffer to remove the buffer of step (d);
(f) concentrating cells obtained in step (e), essentially removing all supernatant and resuspending said cells in a defined volume of physiological buffer; and
(g) determining the number of the cells obtained in step (f), wherein this cell number corresponds to the number of cells of the second cell population contained in the blood sample.

Methods for obtaining human blood samples are known in the art.

The term "non-human antibody" defines in the context of the invention an antibody which is originated from an organism other than human. Preferably, a non-human antibody originates from mice, rat, goat or rabbit.

A corresponding antibody can be, for example, polyclonal or monoclonal. The mentioned derivatives or fragments thereof still retain the binding specificity of the antibody they are derived from. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

The term "antibody, antibody fragment or derivative thereof" also includes embodiments such as chimeric and single chain antibodies, as well as antibody fragments, like, inter alia, Fab fragments. Antibody fragments or derivatives further comprise $F(ab')_2$, Fv or scFv fragments; see, for example, Harlow and Lane (1988) and (1999), loc. cit. Various procedures are known in the art and may be used for the production of such antibodies and/or fragments/derivatives. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals may be used to express antibodies specific for the desired cell surface antigens. Most preferably, the antibody/antibodies is/are (a) monoclonal antibody/antibodies. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of an polypeptide of the invention (Schier, Human Antibodies Hybridomas 7 (1996), 97-105; Malmborg, J. Immunol. Methods 183 (1995), 7-13). It is also envisaged in the context of this invention that the term "antibody, antibody fragment or derivative thereof" comprises antibody constructs which may be expressed in cells, e.g. antibody constructs which may be transfected and/or transduced via, inter alia, viruses or plasmid vectors.

The antibody, antibody fragment or derivative thereof described in the context of the invention is capable to specifically bind/interact with an epitope on the surface of cells contained in a human blood sample. The term "specifically binding/interacting with" as used in accordance with the present invention means that the antibody, antibody fragment or derivative thereof does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest and thus to be antibodies in accordance with this invention. Corresponding methods are described e.g. in Harlow and Lane, 1988 and 1999, loc cit.

The antibody, antibody fragment or derivative thereof specifically binds to/interacts with conformational or continuous epitopes which are unique for the polypeptides or fusion protein of the invention. A conformational or discontinuous epitope is characterized for polypeptide antigens by the presence of two or more discrete amino acid residues which are separated in the primary sequence, but come together on the surface of the molecule when the polypeptide folds into the native protein/antigen to form the epitope (Sela, (1969) Science 166, 1365 and Laver, (1990) Cell 61, 553-6).

The two or more discrete amino acid residues contributing to the epitope are present on separate sections of one or more polypeptide chain(s). In contrast, a continuous or linear epitope consists of two or more discrete amino acid residues which are present in a single linear segment of a polypeptide chain.

The term "conditions that allow labeling of antigens with their cognate antibody" describes conditions that allow a specific binding of the antibody, fragment or derivative thereof with the antigen on the surface of the target cell to occur. Further, under these conditions, a coagulation of the cells in the blood sample is inhibited. These conditions can, for example, be achieved by the additions of suitable agents. Examples for such agents, which can be supplemented to the sample in order to inhibit or avoid the coagulation are described herein below.

The term "density centrifugation" relates in the context of the invention to a well known method of isolating a specific population of cells from a sample by specific physical characteristics of the specific population. Since it is known in the art that different cells of the immune system have inherently differing densities and sizes, one can utilize these parameters to fractionate the individual subpopulations. As cells of differing sizes will have different sedimentation rates, to some extent they can be fractionated by density centrifugation. On the other hand, cells of equivalent size, but differing densities can be fractionated by employing a density gradient centrifugation. By carefully selecting the densities of the media, one can generate highly purified subpopulations of cells from complex mixtures (e.g. lymphocytes).

According to the invention, the density gradient centrifugation is employed to separate the cells of the blood sample. The cells expressing the antigen bound by the at least one antibody, antibody fragment or derivative thereof (respectively, the undesired cell population(s)) accumulate in a pellet. The supernatant consists of the serum phase, the phase of the medium used for the density centrifugation and an interphase between the two phases. In the interphase the erythrocytes (RBC) and the second cell population accumulate. The collection of the supernatant in step (c) of the invention comprises the essential recovery of theses two phases and the interphase.

A "physiological buffer" in accordance with the invention is a buffer with a pH between 7.0 and 7.60 and a salt concentration of approximately 0.85% to 0.9 %. A preferred example of a physiological buffer is phosphate buffered saline (PBS). As stated herein below it is further preferred that PBS is further supplemented with non-human serum. The serum is added in order to cover unspecific binding sites (blocking agent). PBS is preferably supplemented with approximately 5 % non-human serum, more preferably with 3 % non-human serum and most preferably with 2 % non-human serum. Non-limiting examples of non-human serum comprise fetal calf serum (FCS), bovine serum, mouse serum, rat serum or goat serum. It is particularly preferred that the non-human serum is FCS.

It is understood that the number of different non-human antibodies, antibody fragments or derivatives thereof, which may be comprised in the first component of the cocktail recited in step (a) determines the homogeneity or the heterogeneity of the second cell population. In the case, where only one type of antibodies, antibody fragments or derivatives thereof with one specificity is comprised in the first component of the cocktail, only a binding component for a single antigen is contacted with the sample of human blood. The isolated second cell population comprises in this case all types of cells originally comprised in the blood sample except for the erythrocytes and the cell population which expressed the specific antigen of the first-component-antibody, antibody fragment or derivative thereof on the cell surface. As described herein below for a preferred embodiment of the invention, certain cocktails comprising antibodies, antibody fragments or derivatives thereof with different specificities allows the isolation of a homogenous or essentially homologous second cell population (i.e. a population having more than 90%, such as more than 95% or more than 98% or even more than 99% such as 100% of one cell type). This is achieved by selecting antibodies, antibody fragments or derivatives thereof with a specificity for antigens characteristic for each of the (undesired) cell populations comprised in the sample with the exception of the second cell population/cell population of interest.

The person skilled in the art is aware of different alternatives to determine the number of cells in a sample obtained by the method of the invention. Such alternatives comprise approaches using ELISA (e.g. with peroxidase or alkaline phosphatase as active enzyme), assays using colloidal gold, assays measuring a pellet size using a scaled capillary, the manual counting in a hematocytometer (e.g. Neubauer-type) under light microscope, automatic counting in a hematocytometer (e.g. Sysmex-type) or electronic cell-counter (e.g. casy-type). Preferred embodiments of methods to determine the number of cells in a sample are described herein below.

It has surprisingly been found that the method of the invention can be used with a limited requirement of laboratory equipment and reduced costs compared to well known experimental approaches for the determination of the number of cells of a specific cell population in a blood sample. The determination of the number of a selected cell population or the ratio between different selected cell populations is of specific interest in the diagnosis of diseases and for the staging of diseases. A preferred example of a disease which can be analyzed by the method of the invention is a HIV-infection. Today, a record 39.4 million people are infected with HIV, with 95% of all new HIV infections occurring among people in developing countries (UNAIDS 2005). While antiretroviral therapy is becoming more and more affordable and accessible, the laboratory tests necessary for monitoring both the infection and its therapy are not (Balakrishnan *et al.* 2005). The present invention provides a simple manual and low-cost technique for the quantitation of specific cell populations such as CD4$^+$ T-cells in a blood sample taken from human subjects, e.g. HIV-infected individuals. The method of the invention is developed to be operable in resource-limited settings.

The negative-selection-method known in the art (e.g. RosetteSep™), was designed as a qualitative technique for high purity cell enrichment. Until today, it is used only as a qualitative assay (Busch *et al.* 2004; Bischoff *et al.* 2002). According to the manufacturer, this technique shows a very good purity (90 5%) but poor recovery (59 $\pm$ 24%), so that it could not be used as a quantitative assay. Furthermore, the manufacturer recommends to use rather high amounts of blood (1-15 mL), which accordingly require high amounts of antibody cocktail (50 - 750 $\mu$L), thus making the method relatively expensive (5 - 70 € / sample). According to the method of the invention the newly developed "modified negative selection" (MNS) technique provides recovery rates and reduce reagent amounts to the effect that it can be used e.g. as a quantitative assay for the determination of CD4 counts. In one embodiment of the invention the incubation time is increased compared to the protocol of the manufacture from 20 to 30 minutes to ensure a complete depletion of all unwanted cells. To achieve a higher recovery, the entire supernatant is transferred after gradient centrifugation, thus ensuring that all enriched CD4$^+$ T-cells are harvested. Furthermore, a lysing step is added in order to remove remaining RBCs (red blood cells). After the last centrifugation step, the remaining liquid is removed (preferably completely) from the microtube. This may be achieved e.g. by using a cotton bud. The preferred complete removal of the remaining liquid avoids an incorrect dilution when the enriched cells (here CD4$^+$ T-cells) are resuspended.

The MNS-method introduced in this study shows high correlation with flow cytometry ($r$=0.91). The mean difference of 15.35 $\pm$ 12.45% represents an acceptable deviation for the analysis of the amount of specific cell populations in a sample. The recovery rates in the exemplifying embodiment of isolating CD4$^+$ T-cells is further improved by transferring the *entire* supernatant containing the desired CD4$^+$ T-cells into a fresh microtube after gradient centrifugation to harvest *all* CD4$^+$ T-cells. Accordingly, in a further preferred embodiment of the invention essentially the entire supernatant containing the second cell population is collected/recovered in step (c) of the method. The term "essentially entire" describes the collection/recovery of more than 90% of the supernatant, such as more than 95% or more than 98% or even more than 99% such as 100% of the supernatant. This will also apply for other embodiments of the method. The invention allows in a preferred embodiment the harvest of all cells of interest. According to the teaching of the invention it is not profitable to collect enriched cells only from the interphase e.g. between plasma and Ficoll® when working with microtubes and small reagent amounts. Thus, e.g. for the RosetteSep™ the recovery is improved by the method of the invention from originally 59 $\pm$ 24% up to 79.36 $\pm$ 19.13%, purity slightly deteriorated from 90 5% down to 81.10 $\pm$ 10.78%. Without whishing to be bound by theory it is understood that the lower purity is also due to the composition of the commercially available antibody cocktail. It may be possible that this specific cocktail is not suitable to deplete all unwanted cells from any blood sample, particularly from pathological blood (which may comprises e.g. an increased numbers of NK-cells, $\gamma\delta$-lymphocytes, monocytes). The cocktail can be easily be replaced by a different one, better adapted to the purpose of the present invention. Further, it is known in the art that these cells are sometimes hard to distinguish from CD4$^+$ T-cells under a light microscope and could be miscounted as false positive. This aspect explains several other phenomenons:

- recovery rates >100% seen in table 2,
- differences in purity and recovery values between HIV-blood and non-HIV-blood,
- lower correlation in HIV-samples

For the efficiency of the method of the invention it is essential that several steps are carried out with great care. The first one is the transfer of the entire supernatant following gradient centrifugation. The accurate performance of this transfer is of high importance, since it has impact on both purity and recovery: If the supernatant is not transferred completely, the recovery rate will decrease; if the RBC pellet is mixed up, purity will diminish. Another critical step is the removal of the supernatant in step (f) of the method of the invention. The efficiency of the removal can have influence on the recovery of the cells of interest (e.g. the CD4$^+$ T-cells). The cell pellet must not be damaged when drying the pellet, e.g. by use of a cotton bud, otherwise the recovery of the cells of interest may decrease, leading to inaccurate results. Before layering the obtained cells e.g. on an haemocytometer, the sample is favorably vortexed thoroughly to avoid a decrease in recovery.

The "visual gating" during manual counting can be easily carried out by the person skilled in the art. It is preferred that the method of the invention is carried out a short time after the drawing of the human blood sample, preferably within 24 hours after blood drawing, more preferably within 12 hours after blood drawing, even more preferably within 8, 6, 4 or 2 hours.

A further advantage of the method of the invention over methods known in the art results from the fact that only few pieces of equipment are required: e.g. light optical microscope (preferably with a 40x objective), a counting chamber (e.g. a Neubauer type haemocytometer), a microcentrifuge and a vortexer. Through reducing the initial blood volume to only 100 $\mu$L, the required amount of all reagents, above all the antibody cocktail, is also reduced. Thereby, the costs for analysing one sample were minimised. A commercially available vial of antibody cocktail (10mL) costs US $180, 1 L of density medium costs US $60. The total cost of one MNS-assay, including disposable materials such as tubes and tips can be estimated to approximately US $0.50 per sample when working with 5 $\mu$L of cocktail, and US $0.30 when working with 2 $\mu$L of cocktail, respectively.

The term "negative selection" describes in accordance with its meaning in the art a selection approach which results in the accumulation/isolation of cells from a sample by specific depletion of the undesired cells. The negative selection according to the method of the invention allows for a high correlation with flow cytometry ($r$ = 0.91, $p$ <0.0001).

Thus, the MNS-method shows e.g. sufficient accuracy for giving a reliable CD4 count to the attending physician, enabling him to make decisions as to the initiation of ART or opportunistic infection prophylaxis. At a cost of US $0.30 - US $0.50, it is affordable even to patients living in developing countries. Being a reliable, simple and inexpensive CD4$^+$ T-cell enumeration method, it represents a very attractive alternative to flow cytometry, particularly for small laboratories in resource-limited settings. The advantages described for the preferred embodiment of the enumeration of CD4$^+$ T-cells of course also applies for the other embodiments of the method of the invention.

It is also preferred that one or more washing steps are performed during the different steps according to the method of the invention. It is particularly preferred that the method comprises a step (c') subsequent to step (c) and prior to step (d):

(c') washing the cells obtained in step (c) in a physiological buffer.

In a preferred embodiment of the method of the invention said first and second components are complexed by:

(i) forming antibody-protein A complexes, antibody-protein G complexes or antibody-protein L complexes (wherein antibody-protein G complexes are preferred); or
(ii) incubation of the first and second component with an antibody capable of specifically binding to said first and second component.

The protein A, protein G or protein L can be immobilized on a solid carrier. Examples for such solid carriers comprise agarose or sepharose beads (e.g. tetra-sepharose beads).

In the method of the invention the said second cell population (cell population of interest) is selected from the group consisting of the CD4$^+$ cell population, and the CD8$^+$ cell population

It is further preferred that said first component comprises

(i) at least five non-human antibodies, antibody fragments or derivatives thereof, capable of specifically binding to human CD8, CD16, CD19, CD36 and CD56; or
(ii) at least five non-human antibodies, antibody fragments or derivatives thereof, capable of specifically binding to human CD4, CD16, CD19, CD36 and CD56.

In a preferred embodiment of the method the amount of the at least five non-human antibodies, antibody fragments or derivatives thereof is equal in the first component.

It is also preferred in line with the method invention that the antibody, antibody fragment or derivative thereof is capable of specifically binding to the surface of human erythrocytes is

(i) an antibody, antibody fragment or derivative thereof directed against glycophorines (e.g. CD235a, CD235b, CD236, or CD236R);
(ii) an antibody, antibody fragment or derivative thereof directed against Kell antigens (CD238);
(iii) an antibody, antibody fragment or derivative thereof directed against Rhesus antigens (e.g. CD240CE, CD240D, or CD241);
(iv) an antibody, antibody fragment or derivative thereof directed against B-CAM (CD239); or
(v) an antibody, antibody fragment or derivative thereof directed against ICAM-4 (CD242).

In an alternative embodiment the invention relates to a method for diagnosing or staging a disease related to the number of a cell population of interest in blood, the method comprising the steps of the method of the invention for quantifying a cell population of interest, and determining on the basis of the specific number of cells of the second cell population (cell population of interest)

(i) whether the human from which the blood sample was obtained is affected by a disease; and/or
(ii) the characteristic stage of the disease.

The term "staging a disease" describes in the context of the invention the process of determining the developmental stage of a disease. Such determination is important for the determination of a promising administration scheme for one or more suitable pharmaceutical compositions.

It is preferred that the disease which is staged by the method of the invention is an infection with HIV. A classification (i.e. diagnosis) especially of HIV disease can be undertaken for several purposes and should be distinguished from disease staging. Staging is disease classification that aims primarily at making groupings that have different prognosis and can be used in guiding treatment decisions. Stages attempt to classify disease in a progressive sequence from least to most severe, each higher stage having a poorer prognosis or different medical management than the preceding stage. Thus, the progression of a disease e.g. from the different stages of an HIV infection to the manifestation of AIDS can be assessed by the method of the invention. For the staging of an HIV infection the well known criteria of the WHO may be used.

It is preferred that the invention that the cell population of interest is the CD4$^+$ cell population or the CD8$^+$/CD38$^+$ cell population.

In a further preferred embodiment of the method of the invention the blood is blood having been treated with an agent capable of inhibiting coagulation. Examples for corresponding agents comprise, but are not limited to citrate, EDTA and heparin.

It is further preferred for the method of the invention that

(i) in step (a) of the method of the invention, up to 100$\mu$l of blood are treated with 5$\mu$l of the cocktail, and
(ii) after step (a) and prior to step (b) of the method of the invention, the sample is

(a') diluted with 100$\mu$l of physiological buffer and
(a") incubated for at least 30 min at RT.

In line with the method of the invention it is preferred that the density medium is selected from sucrose-based density media or iodixanol. Examples for sucrose-based density media which are particularly preferred comprise Ficoll$^®$ and Percoll$^™$.

It is also preferred for the method of the invention that the liquid capable of selectively lysing erythrocytes in step (d) (which may be a buffered liquid) is selected from the group consiting of BD FACS lysing solution, aqua destillata and NH$_4$Cl-solution. Preferably the NH$_4$Cl-solution is characterized as follows: pH 7,3; 8,29g NH$_4$Cl; 0,037g disodium-EDTA-2-hydrate; 0,839g NaHCO$_3$ or 1,0g KHCO$_3$; H$_2$O aqua destillata ad 1 litre.

The steps of the method may be carried out in any soluble volume of liquid. It is additionally preferred for the method of the invention that the defined volume of step (f) is up to 100$\mu$l.

As described herein above it is preferred for the methods of the invention that the physiological buffer is phosphate buffered saline containing at least 5 % non-human serum. PBS is preferably a solution as follows: pH 7,4; 8,77g NaCl; 1,57g Na$_2$HPO$_4$ x 2H$_2$O; 0,163g KH$_2$PO$_4$; H$_2$O aqua destillata ad 1 litre. Preferred concentrations for FCS have been described herein above. Accordingly, the PBS is preferably supplemented with approximately 5 % non-human serum, more preferably with 3 % non-human serum and most preferably with 2 % non-human serum. Non-limiting examples of non-human serum comprise fetal calf serum (FCS), bovine serum, mouse serum, rat serum or goat serum. It is particularly preferred that the non-human serum is FCS.

In line with the invention a method is preferred, wherein the number of the cells of interest is determined by staining and/or counting the cells. Methods for staining and/or counting the cells comprise but are not limited to approaches using ELISA (e.g. with peroxidase or alkaline phosphatase as active enzyme), immune-chromatographic methods (e.g. using colloidal gold or colloidal carbon), measuring a pellet size using a scaled capillary, the manual counting in a hematocytometer (e.g. Neubauer-type) under light microscope, automatic counting in a hematocytometer (e.g. Sysmex-type) or electronic cell-counter (e.g. casy-type).

It is further preferred that the method of the invention comprises an additional step (f') subsequent to step (f) and prior to step (g):

(f') positively selecting from the second cell population (cell population of interest) a subpopulation expressing a further characteristic antigen on the cell surface by the use of an additional non-human antibody, antibody fragment or derivative thereof specifically binding to said antigen.

The optional additional step (f') allows for the isolation of a subpopulation of cells which, on the one hand expresses the antigen on the cell surface, which was negatively selected in the previous steps of the method and, on the other hand, the further antigen on the cell surface. In order to effect such additional selection the cells obtained in step f (the second cell population) may be incubated e.g. with dyed or paramagnetic beads following the manufactures protocol. These beads are e.g. coated with the additional non-human antibody, antibody fragment or derivative thereof e.g. by the use of a borate buffer or via a polymeric surface. Optionally one or more additional washing step(s) is/are performed prior to step (g). Accordingly, the preferred embodiment allows for the isolation of a cell population which expresses two different antigens on the cell surface (double positive cells).

In line with the invention the additional non-human antibody, antibody fragment or derivative thereof may be immobilized on a solid carrier. Non-limiting examples for solid carriers comprise latex or sepharose or (para)magnetic beads, microtiterplates, and culture dishes.

It is further preferred for the method of the invention that the second cell population (cell population of interest) is the CD8$^+$ cell population, and the additional non-human antibody, antibody fragment or derivative thereof specifically binds CD38. Thus, according to this preferred embodiment of the invention CD8$^+$/CD38$^+$ are isolated from the human blood sample. The determination of the number of the CD8$^+$/CD38$^+$ double positive cells (CD8$^+$/CD38$^+$ counts) is commonly used in the regular antiretroviral therapy as surrogate marker. By decreasing the virus load in the course of a therapy an overactive immune system of a patient is appeased. The activation marker CD38 on CD8$^+$ cytotoxic T cells is down regulated subsequent to a decrease of the virus load. Such a downregulation is an indicator for the reduction to the T cell activation induced by virus infected circulating cells.

Also described is a kit for performing a method according to the invention, the kit comprising

(a) a first component, comprising at least one non-human antibody, antibody fragment or derivative thereof, capable of specifically binding to an antigen expressed on the surface of a first cell population distinct from a second cell population, said second cell population being the cell population of interest and being devoid of said antigen,
(b) a second component, comprising at least one non-human antibody, antibody fragment or derivative thereof, capable of specifically binding to the surface of human erythrocytes;
(c) optionally an agent capable of crosslinking said antibodies; and
(d) instructions for use.

Examples for agents which are capable of crosslinking the recited antibodies, antibody fragments or derivatives thereof comprise, but are not limited to antibodies, antibody fragments or derivatives thereof specific for the constant region of the non-human antibody (such as goat-anti-mouse or rat-anti-mouse antibodies) or agents for a chemical crosslinking of the antibodies.

Preferably the kit is a kit, wherein said first component comprises

(a) at least five non-human antibodies, antibody fragments or derivatives thereof, capable of specifically binding to human CD8, CD16, CD19, CD36 and CD56; and/or
(b) at least five non-human antibodies, antibody fragments or derivatives thereof, capable of specifically binding to human CD4, CD16, CD19, CD36 and CD56.

More preferably, the kit further comprises

(a) an antibody specifically binding proteins expressed on the surface of γδ-lymphocytes and/or monocytes, but not on the surface of CD4$^+$ cells; and/or
(b) an antibody specifically binding proteins expressed on the surface of γδ-lymphocytes and/or monocytes, but not on the surface of CD8$^+$ cells.

**THE FIGURES SHOW:**

**Figure 1: Correlation plot for CD4$^+$ T-cell counts as determined by flow cytometry (FACS) and by the MNS-technique.**
Data on 51 samples obtained from 23 HIV-patients and 28 healthy blood donors. The correlation coefficient *(r)* was 0.91 *(p* <0.0001).The continuous blue line is the regression line, the red dotted line shows 100% correlation.

**Figure 2: Bland-Altman plot for the absolute differences in CD4 counts as determined by flow cytometry and by the MNS-technique,** respectively.
Data on 51 samples obtained from 23 HIV-patients and 28 blood donors. The overall mean difference was 106.5 88.5 cells (median 68 cells, range: 4 - 367 cells; n=51).

**Figure 3: "Visual gating"**
Unwanted cells were visually excluded during manual counting. From left to right: overall picture, lymphocyte, eosinophile granulocyte, red blood cell, debris.

**Figure 4: CD4$^+$ T-cell purity after MNS as determinded by flow** cytometry.
*Left:* The scatter gate (R1) includes all lymphocytes and excludes granulocytes, monocytes, red blood cells and debris.
*Middle:* Histogram plot for the CD4$^+$ purity in the whole sample (no gate). Overall no-gate-purity was 72.56 15.72 %
*Right:* Histogram plot for the CD4$^+$ purity inside the lymphocyte gate (R1). Overall lymphocyte-gate-purity was 81.10 10.79 %.

The invention is illustrated by the following examples but it should be understood that this invention is not limited thereto or thereby.

**Example 1: Materials and methods**

**Study design and clinical protocols**

The study consisted of a dual CD4 T-Lymphocyte enumeration by both the modified negative selection protocol (MNS) and flow cytometry (FCM). It was conducted in the City of Leipzig between August and October 2005 with the main objective of comparing MNS with FCM, particularly in view of precision and cost of both methods. Study participants included 25 patients infected with HIV and 29 healthy donors. Patient age ranged from 20 to 71 years (mean 41.79 15.11 years). The HIV-patients regularly attended the AIDS consultation hours at the Clinic for Dermatology and Venereal Diseases or at the Clinic for Infectious and Tropical Diseases in Leipzig. All were asymptomatic patients with CD4 counts between 96 and 1037 cells/$\mu$L, with or without a history of antiretroviral therapy. The healthy donors were official blood donors with CD4 counts between 429 and 1696 cells/$\mu$L.

Peripheral venous blood was drawn into small Monovettes (Sarstedt, Nümbrecht, Germany,) with ethyl diamine tetraacetate (EDTA) as anticoagulant, and was processed within 8 hours. The study was conducted in a blinded manner and according to the rules of the Ethic Committee on Human Research of the University of Leipzig.

**CD4[+] T-Cell enumeration by flow cytometry**

CD4[+] T-lymphocytes were enumerated for each specimen with a 4-colour, two-platform flow cytometer (FACSCalibur™; Becton Dickinson Immunocytometry Systems, Heidelberg, Germany), following our in-house laboratory instructions. 100 $\mu$L of EDTA-anticoagulated whole peripheral blood were incubated with 2 $\mu$L of anti-CD4-PE (Immunotech; Beckman-Coulter, Krefeld, Germany) for 15 minutes at room temperature (RT). Erythrocytes were removed by incubation with 2 mL of BD Lysing Solution (BD Biosciences, Heidelberg, Germany) for 10 minutes at room temperature. After centrifugation (5 minutes at 250xg, RT), the cells were washed with 3 mL of PBS, centrifuged again (5 minutes at 250xg, RT) and resuspended in 250 $\mu$L of PBS / 1% Formaldehyde (FA). The cells were subsequently sorted for CD4[+] T-cells on FACSCalibur™. Finally, the absolute CD4 count was calculated from the percentage of CD4[+] cells within all lymphocytes as determined by FACSCalibur™ and the TLC obtained by a routine haemocytometer (Sysmex®, Norderstedt, Germany).

**CD4[+] T-cell enumeration by the MNS-method**

Enrichment of CD4[+] T-cells was performed by a modified density-based negative selection protocol (RosetteSep™; CellSystems, St. Katharinen, Germany), which uses a special antibody-cocktail for the enrichment of cells from whole blood. The "RosetteSep™ Human CD4[+] T Cell Enrichment Cocktail" consists of mouse IgG1 antibodies to human lineage antigens (CD8, CD16, CD19, CD36 and CD56), crosslinked to mouse IgG1 anti-human glycophorin antibodies by means of rat anti-mouse IgG1 secondary antibodies, thus forming bi-specific tetrameric antibody complexes (TACs). These TACs crosslink all unwanted nucleated cells (NC) to multiple red blood cells (RBC) by forming RBC rosettes around the targeted NC, thus increasing the density of the unwanted (rosetted) cells, such that they pellet along with the free RBC when centrifuged over a density medium. The desired CD4[+] T-cells remain unlabeled with antibody and can be collected as enriched population at the interface between the plasma and the density medium (StemCell Technologies Inc. 2005).

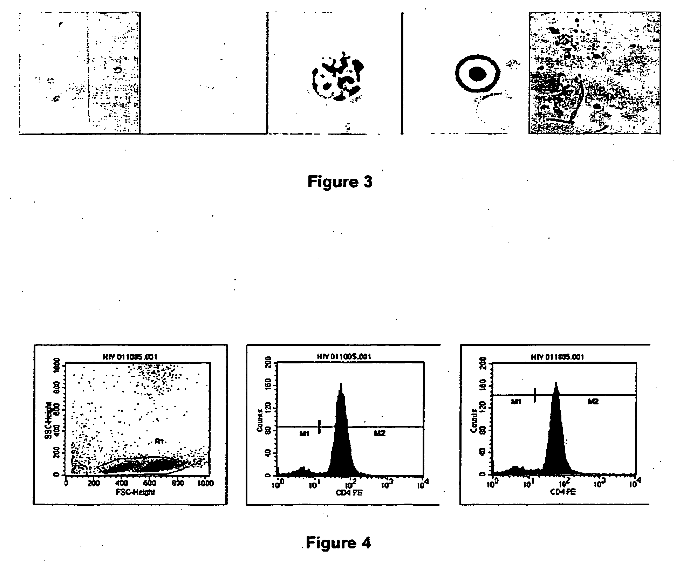

A 1.5 mL microtube was used to add 5 $\mu$L of cocktail to 100 $\mu$L of EDTA-anticoagulated venous whole blood. The sample was diluted with 100 $\mu$L of PBS / 2% FCS, vortexed gently and then rocked for 30 minutes, RT. For gradient centrifugation, the mixture was underlayered with 150 $\mu$L of Ficoll-Paque® PLUS density medium (CellSystems, St. Katharinen, Germany) and centrifuged (1000 x g, 5 min, RT) in a microcentrifuge (Eppendorf, Hamburg, Germany). To ensure that *all* CD4[+] T-cells are harvested, the *entire* supernatant was transferred into a fresh microtube, taking great care not to mix up the RBC pellet containing the unwanted cells. The transferred supernatant was washed with 1 mL of PBS / 2% FCS, centrifuged again (5 minutes at 1200 x g, RT) and incubated with 100$\mu$L of FACS lysing buffer (BD Biosciences, Heidelberg, Germany) to eliminate remaining erythrocytes. The mixture was rocked gently for 10 minutes, RT, followed by washing with 1 mL PBS / 2% FCS and centrifugation (5 minutes at 1200 x g, RT). To obtain a dry cell pellet, *all* remaining liquid was carefully removed from the microtube using a cotton bud, taking care not to damage the cell pellet. Cells were resuspended in 100 $\mu$L PBS / 2% FCS (same amount as the original blood sample). Finally, after thorough vortexing, 10 $\mu$L of the CD4[+] T-cell suspension were layered on a Neubauer type haemocytometer (Feinoptik GmbH, Blankenburg, Germany) and counted under a light optical microscope, 40fold magnification, following strict counting rules that visually exclude all granulocytes, erythrocytes, platelets and debris to ensure that only lymphocytes are counted. In the style of the terminology used in flow cytometry, we called this "visual gating" (see figure 4).

**Analysis of purity and yield**

The purity of each specimen assayed by the MNS-technique was assessed by analyzing the enriched CD4[+] T-cells on FACSCalibur™ flow cytometer (Becton Dickinson Immunocytometry Systems, Heidelberg, Germany) after staining with anti-CD4-PE (Immunotech; Beckman-Coulter, Krefeld, Germany). We assessed two CD4[+] T-cell purity values for each specimen: a) the percentage of CD4[+] cells in a scatter gate that included lymphocytes and excluded granulocytes, monocytes, red blood cells and debris, and b) the percentage of CD4[+] cells of all cells in the sample (no gate; see figure 4, right panel). Since strict rules had been set for manual counting ("visual gating", see above), the CD4[+] purity obtained within the lymphocyte gate during flow cytometry analysis must be considered as the corresponding and therefore significant value. Hence, all further analyses and calculations refer to this purity value.

For assessment of yield (recovery rate), the real number of CD4[+] T-cells in each specimen assayed by MNS was calculated and then expressed as percentage of the number of CD4[+] T-cells obtained by flow cytometry *(FC).*

$$\text{real CD4-count} \quad = \text{manual cell count x gate purity [\%] / 100}$$

$$\text{recovery rate [\%]} \quad = \text{real CD4 count / FC CD4 count x 100}$$

**Analysis of non-CD4[+] T-cells**

The percentages of non-CD4[+] T-cells remaining after MNS were assessed by analyzing a twofold assayed blood sample (1 x with 2 $\mu$L and 1x with 5 $\mu$L of antibody-cocktail) on FACSCalibur™ (Becton-Dickinson, Heidelberg, Germany) after counterstaining with fluorescent dye (Immunotech; Beckman-Coulter, Krefeld, Germany).

Staining 1: CD4-APC, CD3-FITC, CD45-PerCP, CD8-PE
Staining 2: CD3 -FITC, CD16,56-PE, CD45-PerCP, CD 19-APC
Staining 3: CD11 b-FITC, CD4-PE, CD45-PerCP, CD14-APC
Staining 4: CD235a-PE, CD236-FITC, CD4-PE CY5
Staining 5: CD 3-PE, Anti-TCR-$\gamma\delta$-FITC

**Statistical analysis**

The relations between CD4 counts assessed with FCM and MNS, respectively were determined with Bland-Altman-Plot and Passing / Bablok - regression using Medcalc® (Medcalc software, Mariakerke, Belgium). Windows Excel 2000 software was used to analyze and describe all assessed data. The variables analyzed were the number of CD4[+] T - cells, CD4-purity and CD4-recovery. All data were calculated as mean $\pm$ SD. Due to the specialties of diagnostic tests however, data are displayed as median, range and confidence interval.

**Example 2: Results**

**CD4 count range and thresholds**

In the study, fifty-one pairs of values of CD4 counts were generated by both flow cytometry and the MNS-technique from 51 blood samples (23 HIV-infected patients and 28 healthy blood donors). Purity and recovery were analyzed for 47 samples (19 HIV-samples and 28 healthy-donor-samples). The MNS-assay could not be performed with 1 healthydonor-sample and 2 HIV-samples due to shipping problems. Purity analysis could not be performed with another 4 HIV-samples due to technical problems. The overall mean CD4 count was 707 $\pm$ 315 cells / $\mu$L (median: 664, range: 96 - 1696, *n*=51).

**Correlation**

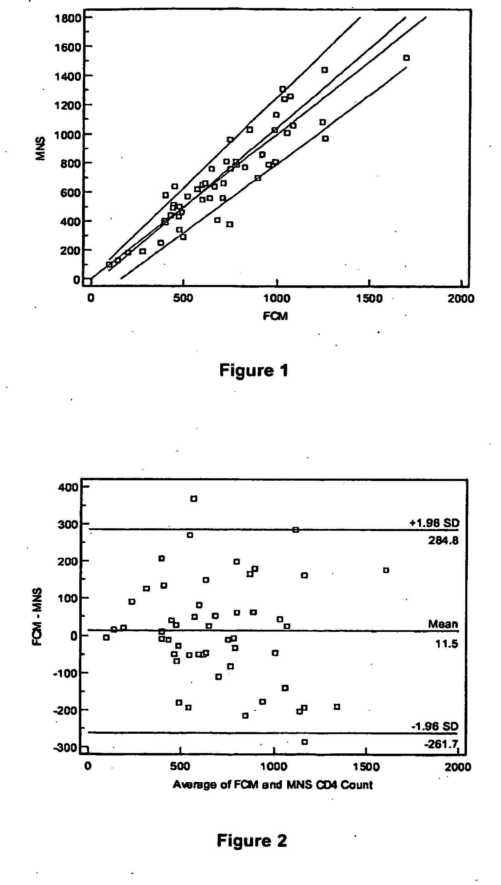

Figure 1 depicts the correlation plot for the two methods for all the data analyzed. The correlation coefficient *(r)* was 0.91 *(p* < 0.000 1) for all the data analyzed, 0.86 (p<0.0001) for HIV-patients and 0.90 (p<0.0001) for non-H IV-patients.

### Difference between the MNS-method and flow cytometry

The absolute mean difference was 106.5 $\pm$ 88.5 cells (median 68 cells, range: 4 - 367 cells; *n*=51; see figure 2). The mean difference in percent was 15.35 $\pm$ 12.45% (median: 11.11%, range: 0.77% - 49.13%; *n*=51).

### Purity and recovery

Purity and recovery values are demonstrated in tables 1 and 2. With healthy donor blood, the original negative selection-method has been reported to yield 90 $\pm$ 5% pure CD4+ T-cells with a recovery of 59 $\pm$ 24% (StemCell Technologies Inc. 2005). With the modified technique, we approached a slightly lower overall purity of 81.10 $\pm$ 10.78% (median: 83.40%, range: 38.64 - 95.55%, *n* = 47; inside lymphocyte gate) and 72.56 $\pm$ 15.72 % (median: 76.82 %, range: 24.97% - 91.11 %, *n* = 47; no gate), respectively. In contrast, overall recovery of CD4+ T-cells could be increased up to 79.36 $\pm$ 19.13% (median: 82.43%, range: 38.94% - 125.27 %, *n* = 47).
With healthy blood donors (*n*=28), CD4+ purity was 84.35 $\pm$ 5.10 % (median: 85.3 8%, range 46.23% - 92.51 %; inside lymphocyte gate) and 76.68 $\pm$ 12.51 % (median: 78,65%, range 27.27% - 90.19%; no gate). With HIV-patients (*n*=19), CD4+ purity was 76.31 $\pm$ 14.53% (median: 79.93%, range 38.64 - 95.55%; inside lymphocyte gate) and 66.50 $\pm$ 17.86% (median: 71.03%, range 24.97% - 91.11 %; no gate).
Recovery of CD4+ T-cells was 86.61% $\pm$ 12.44% (median: 86.25%, range: 60.4 - 111.30%) for healthy donor blood and 68.68 $\pm$ 22.04% (median: 63.47%, range: 38.94% - 125.27%) for HIV-patient blood.

### Analysis of non-CD4+ T-cells

The analysis of the non-CD4+ T-cells remaining after MNS (see table 3) showed that approx. 10% of all cells in the assayed sample were erythrocytes. 3-4% were granulocytes and 2-4% were monocytes. This sums up to 15 - 18% of distinguishable cells. 1-2% were CD8+ T-lymphocytes, 1% were B-lymphocytes, 7% were $\gamma\delta$-T-lymphocytes and 4-7 % were NK-cells, summing up to 13-17% of undistinguishable cells.

### Assessment of cocktail amount

An additional experiment was performed on 3 samples to assess whether similar accuracy could be approached with smaller amounts of the antibody cocktail. Samples exhibiting a median CD4 count of 547 cells/$\mu$L (flow cytometry shows cells were analyzed by the MNS-technique using antibody-cocktail amounts of 2, 3 and 4$\mu$L. No significant difference in the results of CD4+ T-cell enumeration were found when changing the amount of antibody cocktail (data not shown).

### Reproducibility

The reproducibility of the MNS-technique was assessed in a specific set of experiments in which 5 duplicate samples (2 HIV-samples and 3 healthy-donor-samples) were assayed twice using the MNS-technique. The coefficient of variation was 4.05 %.

### Operator-to-operator variability

In another separate experiment, the operator-to-operator variability was analyzed. Five members of staff who had no experience in the MNS-technique were instructed to perform one assay each (all from the same blood sample) by following the modified MNS-protocol. The coefficient of variation was 14.45%. The mean CD4 count as assessed by the 5 staff members was 357 cells/$\mu$L, flow cytometry revealed 352 cells/$\mu$L.
Furthermore an additional experiment with 2 members of staff who had good practice in MNS was performed on 5 samples. The coefficient of variation was 7.19 %.

### Delay in sample handling

The impact of the delay in sample handling was assessed in 5 samples (2 HIV-samples and 3 healthy-donor-samples) that were assayed by the MNS-method at hours 2, 8, 12 and 24 after drawing the blood. The median values were 590 cells/$\mu$L at time 2, 590 cells/$\mu$L at time 8, 560 cells/$\mu$L at time 12 and 640 cells/$\mu$L at hour 24. Mean values were 520 cells/$\mu$L at time 2, 528 cells/$\mu$L at time 8, 520 cells/$\mu$L at time 12 and 604 cells/$\mu$L at hour 24. The mean and median variations within 12h were insignificant.

**Further variations**

The method of the invention can be adjusted for CD4+ quantitation from infant blood, since more and more patients with HIV infection are reported to be children. A first experiment on 4 samples of infant blood showed a correlation with flow cytometry of r = 0.89, (Data not shown). Moreover, as described herein above, the mMNS-method can easily be adapted for the targeting of other cells (e.g. CD8+ or CD19+), and for other immunodiagnostic purposes.

It may be necessary to determine regional specificities of cell compositions in HIV-blood for a further improvement of the cocktail to patient populations in Africa, India and Europe.

**List of tables:**

| | | HIV NG | HIV LG | healthy NG | healthy LG | overall NG | overall LG |
|---|---|---|---|---|---|---|---|
| | 75% quartile | 78.67% | 82.98% | 83.25% | 88.07% | 82.35% | 87.69% |
| | maximum | 91.11% | 95.55% | 90.19% | 92.51% | 91.11% | 95.55% |
| | minimum | 24.97% | 38.64% | 27.27% | 74.11% | 24.97% | 38.64% |
| | 25% quartile | 61.12% | 70.44% | 75.61% | 80.00% | 68.36% | 78.45% |
| | median | 71.03% | 79.93% | 78.65% | 85.38% | 76.82% | 83.40% |

**Table 1. Boxplot CD4+ purity of 47 samples assayed by MNS. LG is for "lymphocyte gate", NG is for "no gate". Since cells were visually gated during manual counting, the CD4+ purity within the lymphocyte gate must be considered as the corresponding and therefore the significant value.**

| | HIV | healthy | overall |
|---|---|---|---|
| 75% quartile | 81.64% | 94.07% | 90.41% |
| maximum | 125.27% | 111.24% | 125.27% |
| minimum | 38.94% | 60.41% | 38.94% |
| 25% quartile | 52.35% | 80.05% | 67.32% |
| median | 63.47% | 86.25% | 82.43% |

**Table. 2. Overall mean CD4$^+$ recovery rates** of 47 samples assayed by MNS was 79.36 ± 19.13% (median: 82.43%, range 38.94% - 125.27%).

**Table 3. The percentages of non-CD4$^+$ T-cells remaining after MNS** were assessed by analyzing a twofold assayed blood sample (1x with 2μL and 1x with 5μL of antibody-cocktail) on FACSCalibur™ after counterstaining with fluorescent dye .

| Marker | cell type | 2μl antibody cocktail | 5μl antibody cocktail |
|---|---|---|---|
| CD8 | cytoxicT-cells | <1% | 2% |
| CD3 | T-lymphocytes | not stained | 7% |
| CD14 | monocytes | 4% | 2% |
| CD11b | granulocytes | 3% | 4% |
| CD 16/56 | NK-cells | 7% | 4% |
| CD19 | B-lymphocytes | 1% | <1 % |
| CD235/236 | RBC | -10% | ~10% |

**REFERENCES**

1. Aidsmap. Monitoring the immune system. www.aidsmap.com. 2005.

2. Balakrishnan P, Dunne M, Kumarasamy N, Crowe S, Subbulakshmi G, Ganesh AK, Cecelia AJ, Roth P, Mayer KH, Thyagarajan SP, Solomon S (2004) An inexpensive, simple, and manual method of CD4 T-cell quantitation in HIV-infected individuals for use in developing countries. Jaids-Journal of Acquired Immune Deficiency Syndromes, 36: 1006-1010.

3. Balakrishnan P, Solomon S, Kumarasamy N, Mayer KH (2005) Low-cost monitoring of HIV infected individuals on highly active antiretroviral therapy (HAART) in developing countries. Indian Journal of Medical Research, 121: 345-355.

4. Bi XQ, Gatanaga H, Tanaka M, Honda M, Ida S, Kimura S, Oka S (2005) Modified Dynabeads method for enumerating CD4(+) T-lymphocyte count for widespread use in resource-limited situations. Jaids-Journal of Acquired Immune Deficiency Syndromes, 38: 1-4.

5. Bischoff FZ, Marquez-Do D, Martinez DI, Horne C, Dang D, Burke A, Lewis DE, Simpson JL (2002) Intact fetal cell isolation from maternal blood using neither MACS nor flow cytometry: Improved isolation using a simple whole blood progenitor cell enrichment approach (RosetteSep (TM)). American Journal of Human Genetics, 71: 563.

6. Busch R, Cesar D, Higuera-Alhino D, Gee T, Hellerstein MK, Mccune JM (2004) Isolation of peripheral blood CD4(+) T cells using RosetteSep (TM) and MACS (TM) for studies of DNA turnover by deuterium labeling. Journal of Immunological Methods, 286: 97-109.

7. Carella AV, Moss MW, Provost V, Quinn TC (1995) A Manual Bead Assay for the Determination of Absolute Cd4 (+) and Cd8(+) Lymphocyte Counts in Human Immunodeficiency Virus-Infected Individuals. Clinical and Diagnostic Laboratory Immunology, 2: 623-625.

8. Chianese R, Nebuloni E, De Paschale M, Gatti A, Mena M (2003) Absolute TCD4(+) counting by a minimalist dual-platform flow cytometric method. Journal of Biological Regulators and Homeostatic Agents, 17: 358-365.

9. Crowe S, Turnbull S, Oelrichs R, Dunne A (2003) Monitoring of human immunodeficiency virus infection in resource-constrained countries. Clinical Infectious Diseases, 37: S25-S35.

10. CyTecs GmbH. New Perspectives for Monitoring HIV-infected Patients in Developing Countries by Affordable CD4+ T-cell Counts. www.cytecs.com. 2005.

11. Diagbouga S, Chazallon C, Kazatchkine MD, de Perre PV, Inwoley A, M'Boup S, David MP, Tenin AT, Soudre R, Aboulker JP, Weiss L (2003) Successful implementation of a low-cost method for enumerating CD4(+) T lymphocytes in resource-limited settings: the ANRS 12-26 study. Aids, 17: 2201-2208.

12. Donnenberg AD, Donnenberg VS (2004) Phenotypic and Functional Measurements on Circulating Immune Cells and their Subsets. In: Lotze MT and Thomson AW, eds. Measuring Immunity: Basic Science and Clinical Practice. Academic Press, 23 7-242.

13. Janossy G, Jani IV, Kahan M, Barnett D, Mandy F, Shapiro H (2002) Precise CD4 T-cell counting using red diode laser excitation: For richer, for poorer. Cytometry, 50: 78-85.

14. Pattanapanyasat K, Lerdwana S, Shain H, Noulsri E, Thepthai C, Prasertsilpa V, Eksaengsri A, Kraisintu K (2003) Low-cost CD4 enumeration in HI V-infected patients in Thailand. Asian Pacific Journal of Allergy and Immunology, 21: 105-113.

15. StemCell Technologies Inc. RosetteSep® for separation of HUMAN cells. https://wwwatemcell.com/product catalog/rosettesep.asp 2005.

16. Storie I, Sawle A, Goodfellow K, Whitby L, Granger V, Reilly JT, Barnett D (2003a) Flow rate calibration I: A novel approach for performing absolute cell counts. Cytometry Part B-Clinical Cytometry, 55B: 1-7.

17. Storie I, Sawle A, Whitby L, Goodfellow K, Granger V, Reilly JT, Barnett D (2003b) Flow rate calibration II: A clinical evaluation study using PanLeucoGating as a single-platform protocol. Cytometry Part B-Clinical Cytometry, 55B: 8-13.

18. U.S.Department of Health and Human Services. Guidelines for the Use of Antiretroviral Agents in HIV- 1-Infected Adults and Adolescents. http://AIDSinfo.nih.gov/guidelines. 2005.

19. UNAIDS. AIDS Epidemic Update 2004. www.unaids.org. 2005.

20. World Health Organization. CD4+ T-cell enumeration technologies. http://www.who.int.2005.

**Claims**

1. A method for quantifying a cell population of interest contained in a human blood sample, comprising the steps of:

    (a) contacting a sample of human blood with a cocktail comprising

        (i) a first component, comprising at least one non-human antibody, antibody fragment or derivative thereof, capable of specifically binding to an antigen expressed on the surface of a first cell population distinct from a second cell population, said second cell population being the cell population of interest and being devoid of said antigen, wherein said second cell population is selected from the group consisting of the CD4$^+$ cell population and the CD8$^+$ cell population, complexed to
        (ii) a second component, comprising at least one non-human antibody, antibody fragment or derivative thereof, capable of specifically binding to the surface of human erythrocytes;
        wherein the contacting is performed under conditions that allow labelling of antigens with their cognate antibody;

    (b) separating antibody-labelled from unlabelled cells contained in the blood sample by means of density centrifugation wherein the minimal degree of density of the medium is about 1.077 g/mL;
    (c) collecting the entire supernatant comprising the second cell population;
    (d) concentrating the second cell population contained in the supernatant and resuspending said cell population in a liquid capable of selectively lysing erythrocytes;
    (e) washing cells obtained in step (d) in a physiological buffer to remove the buffer of step (d);
    (f) concentrating cells obtained in step (e), essentially removing all supernatant and resuspending said cells in a defined volume of physiological buffer; and
    (g) determining the number of the cells obtained in step (f), wherein this cell number corresponds to the number of cells of the second cell population contained in the blood sample.

2. The method of claim 1, additionally comprising a step (c') subsequent to step (c) and prior to step (d):

    (c') washing the cells obtained in step (c) in a physiological buffer.

3. The method of claim 1 or 2, wherein said first and second components are complexed by

    (i) forming antibody-protein A complexes; or
    (ii) incubation of the first and second component with an antibody capable of specifically binding to said first and second component.

4. The method of any one of claims 1 to 3, wherein said first component comprises

    (i) at least five non-human antibodies, antibody fragments or derivatives thereof, capable of specifically binding to human CD8, CD16, CD19, CD36 and CD56; or .
    (ii) at least five non-human antibodies, antibody fragments or derivatives thereof, capable of specifically binding to human CD4, CD16, CD19, CD36 and CD56.

5. The method of any one of claims 1 to 4, wherein the antibody, antibody fragment or derivative thereof that is capable of specifically binding to the surface of human erythrocytes is

    (i) an antibody, antibody fragment or derivative thereof directed against glycophorines;
    (ii) an antibody, antibody fragment or derivative thereof directed against Kell antigens;
    (iii) an antibody, antibody fragment or derivative thereof directed against Rhesus antigens;
    (iv) an antibody, antibody fragment or derivative thereof directed against B-CAM; or
    (v) an antibody, antibody fragment or derivative thereof directed against ICAM-4.

6. A method for diagnosing or staging a disease related to the number of a cell population of interest in blood, comprising the steps of the method of any one of claims 1 to 5 and determining on the basis of the specific number of cells of the second cell population (cell population of interest)

    (i) whether the human from which the blood sample was obtained is affected by a disease; and/or

(ii) the characteristic stage of the disease.

7. The method of claim 6, wherein said disease is an infection with HIV.

8. The method according to claim 7, wherein the cell population of interest is the CD4$^+$ cell population or the CD8$^+$/CD38$^+$ cell population.

9. The method of any one of claims 1 to 8, wherein the blood is blood having been treated with an agent capable of inhibiting coagulation.

10. The method of any one of claims 1 to 9, wherein

(i) in step (a) of claim 1, up to 100$\mu$l of blood are treated with 5$\mu$l of the cocktail, and
(ii) after step (a) and prior to step (b) of claim 1, the sample is

(a') diluted with 100$\mu$l of physiological buffer and
(a") incubated for at least 30 min at RT.

11. The method of any one of claims 1 to 10, wherein the density medium is selected from sucrose-based density media or iodixanol.

12. The method of any one of claims 1 to 11, wherein the liquid capable of selectively lysing erythrocytes in step (d) is selected from the group consisting of BD FACS lysing solution, aqua destillata and NH$_4$Cl-solution.

13. The method of any one of claims 1 to 12, wherein the defined volume of step (f) of claim 1 is up to 100$\mu$l.

14. The method of any one of claims 1 to 13, wherein the physiological buffer is phosphate buffered saline containing at least 5 % non-human serum.

15. The method of any one of claims 1 to 14, wherein the number of the cells of interest is determined by staining and/or counting the cells.

16. The method of any one of claims 1 to 15 comprising an additional step (f) subsequent to step (f) and prior to step (g):

(f') positively selecting from the second cell population a subpopulation expressing a further characteristic antigen on the cell surface by the use of an additional non-human antibody, antibody fragment or derivative thereof specifically binding to said further antigen.

17. The method of claim 16, wherein the second cell population is the CD8$^+$ cell population, and the additional non-human antibody, antibody fragment or derivative thereof specifically binds CD38.

**Patentansprüche**

1. Verfahren zur Quantifizierung einer Zellpopulation von Interesse, die in einer menschlichen Blutprobe enthalten ist, das folgende Schritte umfasst:

(a) Inkontaktbringen einer Probe menschlichen Bluts mit einem Gemisch, das umfasst:

(i) einen ersten Bestandteil, der zumindest einen nicht-menschlichen Antikörper, ein Antikörperfragment oder ein Derivat davon umfasst, der/das zur spezifischen Bindung an ein Antigen fähig ist, das an der Oberfläche einer ersten Zellpopulation, die unterschiedlich zu einer zweiten Zellpopulation ist, exprimiert wird, wobei die zweite Zellpopulation die Zellpopulation von Interesse ist und frei von dem Antigen ist, wobei die zweite Zellpopulation aus der Gruppe ausgewählt ist, die aus der CD4$^+$-Zellpopulation und der CD8$^+$-Zellpopulation besteht, der komplexiert ist mit
(ii) einem zweiten Bestandteil, der zumindest einen nicht-menschlichen Antikörper, ein Antikörperfragment oder ein Derivat davon umfasst, der/das zur spezifischen Bindung an die Oberfläche menschlicher Erythrozyten fähig ist;

wobei der Kontakt unter Bedingungen stattfindet, die die Markierung der Antigene mit ihrem erkennenden Antikörper erlauben;

(b) das Abtrennen Antikörper-markierter von unmarkierten Zellen, die in der Blutprobe enthalten sind, mittels Dichtezentrifugation, wobei der minimale Dichtegrad des Mediums etwa 1,077 g/mL ist;

(c) das Sammeln des gesamten Überstands, der die zweite Zellpopulation umfasst;

(d) das Konzentrieren der zweiten Zellpopulation, die im Überstand enthalten ist, und das Resuspendieren der Zellpopulation in einer Flüssigkeit, die zur selektiven Lyse von Erythrozyten fähig ist,

(e) das Waschen der im Schritt (d) gewonnenen Zellen in einem physiologischen Puffer, um den Puffer aus Schritt (d) zu entfernen;

(f) das Konzentrieren der in Schritt (e) gewonnenen Zellen, wobei im Wesentlichen der gesamte Überstand entfernt wird und die Zellen in einem definiertem Volumen an physiologischem Puffer resuspendiert werden; und

(g) das Bestimmen der Anzahl an Zellen, die im Schritt (f) gewonnen wurden, wobei diese Zellanzahl der Anzahl an Zellen der zweiten Zellpopulation entspricht, die in der Blutprobe enthalten ist.

2. Verfahren nach Anspruch 1, das zusätzlich den Schritt (c') umfasst, der auf den Schritt (c) folgt und vor dem Schritt (d) stattfindet:

(c') Waschen der Zellen, die im Schritt (c) gewonnen wurden, in einem physiologischen Puffer.

3. Verfahren nach Anspruch 1 oder 2, wobei der erste und zweite Bestandteil komplexiert werden durch

(i) die Bildung von Antikörper-Protein A-Komplexen; oder

(ii) die Inkubation des ersten und zweiten Bestandteils mit einem Antikörper, der zur spezifischen Bindung an den ersten und zweiten Bestandteil fähig ist.

4. Verfahren nach einem der Ansprüche 1 bis 3, wobei der erste Bestandteil umfasst:

(i) zumindest fünf nicht-menschliche Antikörper, Antikörperfragmente oder Derivate davon, die zur spezifischen Bindung an menschliches CD8, CD16, CD19, CD36 und CD56 fähig sind; oder

(ii) zumindest fünf nicht-menschliche Antikörper, Antikörperfragmente oder Derivate davon, die zur spezifischen Bindung an menschliches CD4, CD16, CD19, CD36 und CD56 fähig sind.

5. Verfahren nach einem der Ansprüche 1 bis 4, wobei der Antikörper, das Antikörperfragment oder das Derivat davon, der/das zur spezifischen Bindung an die Oberfläche von menschlichen Erythrozyten fähig ist,

(i) ein Antikörper, Antikörperfragment oder Derivat davon ist, der/das gegen Glycophorine gerichtet ist;

(ii) ein Antikörper, Antikörperfragment oder Derivat davon ist, der/das gegen Kell-Antigene gerichtet ist;

(iii) ein Antikörper, Antikörperfragment oder Derivat davon ist, der gegen Rhesus-Antigene gerichtet ist;

(iv) ein Antikörper, Antikörperfragment oder Derivat davon ist, der/das gegen B-CAM gerichtet ist; oder

(v) ein Antikörper, Antikörperfragment oder Derivat davon ist, der/das gegen ICAM-4 gerichtet ist.

6. Verfahren zur Diagnose oder zur Bestimmung des Stadiums einer Krankheit, die mit der Anzahl einer Zellpopulation von Interesse im Blut in Beziehung steht, das die Schritte des Verfahrens nach einem der Ansprüche 1 bis 5 umfasst und die Bestimmung, basierend auf der spezifischen Anzahl an Zellen der zweiten Zellpopulation (Zellpopulation von Interesse),

(i) ob der Mensch, von dem die Blutprobe entnommen wurde, von der Krankheit betroffen ist; und/oder

(ii) des charakteristischen Stadiums der Krankheit.

7. Verfahren nach Anspruch 6, wobei die Krankheit eine Infektion mit HIV ist.

8. Verfahren nach Anspruch 7, wobei die Zellpopulation von Interesse die CD4$^+$-Zellpopulation oder die CD8$^+$/CD38$^+$-Zellpopulation ist.

9. Verfahren nach einem der Ansprüche 1 bis 8, wobei das Blut Blut ist, das mit einem Agens behandelt wurde, das zur Hemmung der Koagulation fähig ist.

**10.** Verfahren nach einem der Ansprüche 1 bis 9, wobei

(i) im Schritt (a) des Anspruchs 1 bis zu 100μl Blut mit 5μl des Gemischs behandelt werden, und
(ii) nach dem Schritt (a) und vor dem Schritt (b) des Anspruchs 1, die Probe

(a') mit 100μl an physiologischem Puffer verdünnt wird und
(a'') zumindest 30 min bei RT inkubiert wird.

**11.** Verfahren nach einem der Ansprüche 1 bis 10, wobei das Dichtemedium aus auf Saccharose basierenden Dichtemedien oder Iodixanol ausgewählt wird.

**12.** Verfahren nach einem der Ansprüche 1 bis 11, wobei die Flüssigkeit, die zur selektiven Lyse von Erythrozyten im Schritt (d) fähig ist, aus der Gruppe ausgewählt wird, die aus BD FACS-Lyselösung, destilliertem Wasser und $NH_4Cl$-Lösung besteht.

**13.** Verfahren nach einem der Ansprüche 1 bis 12, wobei das definierte Volumen des Schritts (f) des Anspruchs 1 bis zu 100μl ist.

**14.** Verfahren nach einem der Ansprüche 1 bis 13, wobei der physiologische Puffer eine phosphatgepufferte Salzlösung ist, die zumindest 5% nicht-menschliches Serum enthält.

**15.** Verfahren nach einem der Ansprüche 1 bis 14, wobei die Anzahl an Zellen von Interesse durch Färben und/oder Zählen der Zellen ermittelt wird.

**16.** Verfahren nach einem der Ansprüche 1 bis 15, das einen zusätzlichen Schritt (f') umfasst, der auf den Schritt (f) folgt und vor dem Schritt (g) stattfindet:

(f') das positive Selektieren einer Subpopulation aus der zweiten Zellpopulation, die ein weiteres charakteristisches Antigen an der Zelloberfläche exprimiert, mittels eines zusätzlichen nicht-menschlichen Antikörpers, Antikörperfragments oder Derivats davon, der/das spezifisch an das weitere Antigen bindet.

**17.** Verfahren nach Anspruch 16, wobei die zweite Zellpopulation die CD8$^+$-Zellpopulation ist und der/das zusätzliche nicht-menschliche Antikörper, Antikörperfragment oder Derivat davon spezifisch CD38 bindet.

**Revendications**

**1.** Procédé de quantification d'une population cellulaire d'intérêt contenue dans un échantillon de sang humain, comprenant les étapes consistant à :

(a) mettre en contact un échantillon de sang humain avec un cocktail comprenant

(i) un premier composant, comprenant au moins un anticorps non humain, un fragment d'anticorps ou un dérivé de celui-ci, capable de se lier spécifiquement à un antigène exprimé sur la surface d'une première population cellulaire distincte d'une seconde population cellulaire, ladite seconde population cellulaire étant la population cellulaire d'intérêt et étant dépourvue dudit antigène, où la seconde population cellulaire est choisie dans le groupe constitué de la population des cellules CD4$^+$ et de la population des cellules CD8$^+$, complexé à
(ii) un second composant, comprenant au moins un anticorps non humain, un fragment d'anticorps ou un dérivé de celui-ci, capable de se lier spécifiquement à la surface des érythrocytes humains ;

où la mise en contact est réalisée dans des conditions qui permettent le marquage des antigènes avec leur anticorps apparenté ;
(b) séparer les cellules marquées par l'anticorps de celles non marquées contenues dans l'échantillon de sang au moyen d'une centrifugation en gradient de densité où le degré minimal de densité du milieu est d'environ 1,077 g/ml ;
(c) recueillir le surnageant entier comprenant la seconde population cellulaire ;
(d) concentrer la seconde population cellulaire contenue dans le surnageant et remettre en suspension ladite population cellulaire dans un liquide capable de lyser sélectivement les érythrocytes ;

(e) laver les cellules obtenues dans l'étape (d) dans un tampon physiologique pour éliminer le tampon de l'étape (d) ;

(f) concentrer les cellules obtenues dans l'étape (e), éliminer pratiquement la totalité du surnageant et remettre en suspension lesdites cellules dans un volume défini de tampon physiologique ; et

(g) déterminer le nombre des cellules obtenues dans l'étape (f), où ce nombre de cellules correspond au nombre de cellules de la seconde population cellulaire contenue dans l'échantillon de sang.

2. Procédé selon la revendication 1, comprenant en outre une étape (c') postérieure à l'étape (c) et antérieure à l'étape (d) :

(c') laver les cellules obtenues dans l'étape (c) dans un tampon physiologique.

3. Procédé selon la revendication 1 ou 2, dans lequel lesdits premier et second composants sont complexés par

(i) la formation de complexes anticorps-protéine A ; ou

(ii) l'incubation du premier et du second composant avec un anticorps capable de se lier spécifiquement audit premier et second composant.

4. Procédé selon l'une quelconque des revendications 1 à 3, dans lequel ledit premier composant comprend

(i) au moins cinq anticorps non humains, fragments d'anticorps ou dérivés de ceux-ci, capables de se lier spécifiquement aux CD8, CD16, CD19, CD36 et CD56 humains ; ou

(ii) au moins cinq anticorps non humains, fragments d'anticorps ou dérivés de ceux-ci, capables de se lier spécifiquement aux CD4, CD16, CD19, CD36 et CD56 humains.

5. Procédé selon l'une quelconque des revendications 1 à 4, dans lequel l'anticorps, le fragment d'anticorps ou le dérivé de celui-ci qui est capable de se lier spécifiquement à la surface des érythrocytes humains est

(i) un anticorps, un fragment d'anticorps ou un dérivé de celui-ci dirigé contre des glycophorines ;

(ii) un anticorps, un fragment d'anticorps ou un dérivé de celui-ci dirigé contre des antigènes Kell ;

(iii) un anticorps, un fragment d'anticorps ou un dérivé de celui-ci dirigé contre des antigènes Rhésus ;

(iv) un anticorps, un fragment d'anticorps ou un dérivé de celui-ci dirigé contre B-CAM ; ou

(v) un anticorps, un fragment d'anticorps ou un dérivé de celui-ci dirigé contre ICAM-4.

6. Procédé de diagnostic ou de détermination du stade d'une maladie lié au nombre de cellules d'une population cellulaire d'intérêt dans le sang, comprenant les étapes du procédé selon l'une quelconque des revendications 1 à 5 et la détermination sur la base du nombre spécifique de cellules de la seconde population cellulaire (population cellulaire d'intérêt)

(i) si l'humain à partir duquel l'échantillon de sang a été obtenu est affecté par une maladie ; et/ou

(ii) du stade caractéristique de la maladie.

7. Procédé selon la revendication 6, dans lequel ladite maladie est une infection par le VIH.

8. Procédé selon la revendication 7, dans lequel la population cellulaire d'intérêt est la population de cellules CD4$^+$ ou la population de cellules CD8$^+$/CD38$^+$.

9. Procédé selon l'une quelconque des revendications 1 à 8, dans lequel le sang est du sang ayant été traité avec un agent capable d'inhiber la coagulation.

10. Procédé selon l'une quelconque des revendications 1 à 9, dans lequel

(i) dans l'étape (a) selon la revendication 1, jusqu'à 100 μl de sang sont traités avec 5 μl du cocktail, et

(ii) après l'étape (a) et avant l'étape (b) selon la revendication 1, l'échantillon est

(a') dilué avec 100 μl de tampon physiologique et

(a") incubé pendant au moins 30 min à température ambiante.

EP 1 852 702 B1

**11.** Procédé selon l'une quelconque des revendications 1 à 10, dans lequel le milieu à densité est choisi parmi des milieux à densité à base de saccharose ou de l'iodixanol.

**12.** Procédé selon l'une quelconque des revendications 1 à 11, dans lequel le liquide capable de lyser sélectivement les érythrocytes dans l'étape (d) est choisi dans le groupe constitué de la solution lytique BD FACS, d'eau distillée et de solution de $NH_4Cl$.

**13.** Procédé selon l'une quelconque des revendications 1 à 12, dans lequel le volume défini de l'étape (f) selon la revendication 1 est jusqu'à 100 µl.

**14.** Procédé selon l'une quelconque des revendications 1 à 13, dans lequel le tampon physiologique est le tampon phosphate salin contenant au moins 5 % de sérum non humain.

**15.** Procédé selon l'une quelconque des revendications 1 à 14, dans lequel le nombre des cellules d'intérêt est déterminé par coloration et/ou comptage des cellules.

**16.** Procédé selon l'une quelconque des revendications 1 à 15, comprenant une étape supplémentaire (f) postérieure à l'étape (f) et antérieure à l'étape (g) :

(f) sélectionner positivement à partir de la seconde population cellulaire une sous-population exprimant un autre antigène caractéristique sur la surface cellulaire par l'utilisation d'un anticorps non humain supplémentaire, d'un fragment d'anticorps ou d'un dérivé de celui-ci se liant spécifiquement audit autre antigène.

**17.** Procédé selon la revendication 16, dans lequel la seconde population cellulaire est la population des cellules CD8[+] et l'anticorps non humain supplémentaire, le fragment d'anticorps ou le dérivé de celui-ci se lie spécifiquement à CD38.

Figure 1

Figure 2

**Figure 3**

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4946778 A

### Non-patent literature cited in the description

- **Harlow ; Lane.** Antibodies, A Laboratory Manual. Cold Spring Harbor Laboratory Press, 1988
- **Harlow ; Lane.** Using Antibodies: A Laboratory Manual. Cold Spring Harbor Laboratory Press, 1999
- **Köhler ; Milstein.** *Nature,* 1975, vol. 256, 495-497
- **Kozbor.** *Immunology Today,* 1983, vol. 4, 72
- **Cole et al.** Monoclonal Antibodies and Cancer Therapy. Alan R. Liss, Inc, 1985, 77-96
- **Schier.** *Human Antibodies Hybridomas,* 1996, vol. 7, 97-105
- **Malmborg.** *J. Immunol. Methods,* 1995, vol. 183, 7-13
- **Sela.** *Science,* 1969, vol. 166, 1365
- **Laver.** *Cell,* 1990, vol. 61, 553-6
- *Aidsmap,* 2005, www.aidsmap.com.
- **Balakrishnan P ; Dunne M ; Kumarasamy N ; Crowe S ; Subbulakshmi G ; Ganesh AK ; Cecelia AJ ; Roth P ; Mayer KH ; Thyagarajan SP.** An inexpensive, simple, and manual method of CD4 T-cell quantitation in HIV-infected individuals for use in developing countries. *Jaids-Journal of Acquired Immune Deficiency Syndromes,* 2004, vol. 36, 1006-1010
- **Balakrishnan P ; Solomon S ; Kumarasamy N ; Mayer KH.** Low-cost monitoring of HIV infected individuals on highly active antiretroviral therapy (HAART) in developing countries. *Indian Journal of Medical Research,* 2005, vol. 121, 345-355
- **Bi XQ ; Gatanaga H ; Tanaka M ; Honda M ; Ida S ; Kimura S ; Oka S.** Modified Dynabeads method for enumerating CD4(+) T-lymphocyte count for widespread use in resource-limited situations. *Jaids-Journal of Acquired Immune Deficiency Syndromes,* 2005, vol. 38, 1-4
- **Bischoff FZ ; Marquez-Do D ; Martinez DI ; Horne C ; Dang D ; Burke A ; Lewis DE ; Simpson JL.** Intact fetal cell isolation from maternal blood using neither MACS nor flow cytometry: Improved isolation using a simple whole blood progenitor cell enrichment approach (RosetteSep (TM)). *American Journal of Human Genetics,* 2002, vol. 71, 563
- **Busch R ; Cesar D ; Higuera-Alhino D ; Gee T ; Hellerstein MK ; Mccune JM.** Isolation of peripheral blood CD4(+) T cells using RosetteSep (TM) and MACS (TM) for studies of DNA turnover by deuterium labeling. *Journal of Immunological Methods,* 2004, vol. 286, 97-109
- **Carella AV ; Moss MW ; Provost V ; Quinn TC.** A Manual Bead Assay for the Determination of Absolute Cd4(+) and Cd8(+) Lymphocyte Counts in Human Immunodeficiency Virus-Infected Individuals. *Clinical and Diagnostic Laboratory Immunology,* 1995, vol. 2, 623-625
- **Chianese R ; Nebuloni E ; De Paschale M ; Gatti A ; Mena M.** Absolute TCD4(+) counting by a minimalist dual-platform flow cytometric method. *Journal of Biological Regulators and Homeostatic Agents,* 2003, vol. 17, 358-365
- **Crowe S ; Turnbull S ; Oelrichs R ; Dunne A.** Monitoring of human immunodeficiency virus infection in resource-constrained countries. *Clinical Infectious Diseases,* 2003, vol. 37, S25-S35
- CyTecs GmbH. *New Perspectives for Monitoring HIV-infected Patients in Developing Countries by Affordable CD4+ T-cell Counts,* 2005
- **Diagbouga S ; Chazallon C ; Kazatchkine MD ; de Perre PV ; Inwoley A ; M'Boup S ; David MP ; Tenin AT ; Soudre R ; Aboulker JP.** Successful implementation of a low-cost method for enumerating CD4(+) T lymphocytes in resource-limited settings: the ANRS 12-26 study. *Aids,* 2003, vol. 17, 2201-2208
- **Donnenberg AD ; Donnenberg VS.** Phenotypic and Functional Measurements on Circulating Immune Cells and their Subsets. *Measuring Immunity: Basic Science and Clinical Practice,* 2004, vol. 23, 7-242
- **Janossy G ; Jani IV ; Kahan M ; Barnett D ; Mandy F ; Shapiro H.** Precise CD4 T-cell counting using red diode laser excitation: For richer, for poorer. *Cytometry,* vol. 50, 78-85

- **Pattanapanyasat K ; Lerdwana S ; Shain H ; Noulsri E ; Thepthai C ; Prasertsilpa V ; Eksaengsri A ; Kraisintu K.** Low-cost CD4 enumeration in HIV-infected patients in Thailand. *Asian Pacific Journal of Allergy and Immunology,* 2003
- RosetteSep® for separation of HUMAN cells. Stem-Cell Technologies Inc,
- **Storie I ; Sawle A ; Goodfellow K ; Whitby L ; Granger V ; Reilly JT ; Barnett D.** Flow rate calibration I: A novel approach for performing absolute cell counts. *Cytometry Part B-Clinical Cytometry,* 2003, vol. 55B, 1-7
- **Storie I ; Sawle A ; Whitby L ; Goodfellow K ; Granger V ; Reilly JT ; Barnett D.** Flow rate calibration II: A clinical evaluation study using PanLeuco-Gating as a single-platform protocol. *Cytometry Part B-Clinical Cytometry,* 2003, vol. 55B, 8-13
- *U.S.Department of Health and Human Services. Guidelines for the Use of Antiretroviral Agents in HIV-1-Infected Adults and Adolescents,* 2005, http://AIDSinfo.nih.gov/guidelines
- *UNAIDS,* 2004, mic Update 2004. www.unaids.org.
- CD4+ T-cell enumeration technologies. World Health Organization, 2005